# EUROPEAN PATENT SPECIFICATION

(11) **EP 4 309 706 B1**
(45) Date of publication and mention of the grant of the patent: **02.09.2026**
(21) Application number: 22306088.0
(22) Date of filing: 21.07.2022
(51) Int. Cl.: A61M 5/315

(54) **UNIVERSAL PLUNGER ROD AND METHOD FOR CONNECTING AND DISCONNECTING THE PLUNGER ROD AND A PLUNGER STOPPER**
UNIVERSELLE KOLBENSTANGE UND VERFAHREN ZUM VERBINDEN UND TRENNEN DER KOLBENSTANGE UND EINES KOLBENSTOPPERS
TIGE DE PISTON UNIVERSELLE ET PROCÉDÉ DE CONNEXION ET DE DÉCONNEXION DE LA TIGE DE PISTON ET BUTÉE DE PISTON

(43) Date of publication of application: 24.01.2024
(73) Proprietor: Becton Dickinson France, 38800 Le Pont-de-Claix (FR)
(72) Inventor: GRILLET, Nastasja, 74540 Cusy (FR); GAGLIANO, Julien, 38000 Grenoble (FR); MILLERET, Anne, 38530 Pontcharra (FR)
(74) Representative: Germain Maureau

(56) References cited:
- WO-A1-2012/136570
- AU-A- 2 891 071
- US-B2- 7 798 377
- US-B2- 9 610 400

## Description

The present invention relates to a plunger rod intended to be connected to a plunger stopper in view of expelling or reconstituting a product, such as drug, present within a container of an injection device.

In this application, the distal end of a component or of a device means the end furthest away from the hand of the user and the proximal end means the end closest to the hand of the user, when the component or device is in the use position. Similarly, in this application, the terms "in the distal direction" and "distally" mean in the direction of the injection, and the terms "in the proximal direction" and "proximally" mean in the direction opposite to the direction of injection.

Injection devices, such as syringes, are well known. Many different types of injection devices have been designed for administering medicines. Injection devices usually comprise a container intended to receive the medicines or product to be injected and a plunger rod intended to move a plunger stopper within the container. The plunger stopper may be moved in the distal direction to expel a product at the time of injection. Alternatively, the plunger stopper may be moved in the proximal direction to withdraw a product for example from a separate vial and transfer it within the container of the injection device. Empty and pre-filled disposable injection devices exist on the market, and are convenient, safe and efficient and may be used directly in emergency cases.

Prefilled injections devices are filled with a medicine, such as a pharmaceutical drug, packaged for use, and then typically stored at a doctor's office, hospital, etc. until they are needed for use. In order to ensure that the product present within the container will not leak outwards during the storage, the container is closed at its distal end by a rubber cap, and at its proximal end by a plunger stopper.

In some cases, a plunger rod is connected to the plunger stopper of the prefilled injection device. Nevertheless, in such a condition, the prefilled injection device will take up a significant amount of storage space.

WO2012/136570 describes a release mechanism for a piston/piston rod coupling, to automatically release the piston rod from the piston when the piston reaches a predefined position relative to the reservoir wall. AU2891071 describes an injection syringe which is also equipped with a release mechanism to release the piston rod from the piston when the release mechanism is activated.

Document US11260179 B2 describes a syringe with a plunger rod assembly for connection with a piston seal. The plunger rod assembly has an outer body rod with a central lumen and a flexible expandable flanges at its distal end. The plunger rod assembly further has a central inner rod, which passes through the central lumen of the outer body rod between two positions within the outer body rod - proximal position and distal position. In the distal position, a distal end in form of an annular frontal lip of the central inner rod pushes the flexible expandable flanges outwardly to lock them inside the piston seal. When the central inner rod is pulled backward into the proximal position, the annular frontal lip is not in contact with the flexible expandable flanges and the flexible expandable flanges disengage from the piston seal.

Required storage space is an important feature for prefilled injection devices. It may be especially important when the medicine contained in these devices must be stored and transported at low temperatures.

In hospitals and pharmacies, storage space for medicines is limited. It is thus desirable that the prefilled injection devices occupy as less volume as possible during transportation and/or storage.

In this view, prefilled injection devices are more and more provided without any plunger rod, the proximal end of the injection device being sealed by the plunger stopper on its own. In such cases, separate plunger rods are provided, and the plunger stopper is usually provided with connecting means intended to cooperate with complementary connecting means located on the plunger rod, in order to connect the plunger stopper and the plunger rod together when time has come to proceed with the injection. In a conventional manner, these connecting means are cooperating threads or hooks: the proximal end of the plunger stopper is provided with a cavity having an inner thread and the distal end of the plunger rod is provided with an outer thread capable of cooperating with the inner thread of the cavity of the plunger stopper in order to screw the plunger rod into the plunger stopper.

Anyway, with the development of medicines and of injection device manufacturers all over the world, the designs and dimensions of the inner threads of the plunger stoppers as well as the material forming the plunger stopper may vary and it may happen that some plunger rods are not compatible with the plunger stoppers sealing the proximal ends of prefilled injection devices. The incompatibility between the plunger rod and the plunger stopper may cause bending and/or breakage of the plunger rod during the screwing step of the plunger rod into the plunger stopper and/or may impact container closure integrity and the injection of the product cannot take place in a safe manner. Also, for two not compatible plunger stopper and plunger rods, withdrawal may be impossible and reconstitution cannot be executed properly.

There is therefore a need for a plunger rod that would be compatible with any plunger stopper, in such a way that the user would feel confident that the connection between the plunger rod and the plunger stopper is effective and secured, whatever the specific feature, the design and/or the dimensions of the connecting means, such as the inner thread or hook space, of the cavity of the plunger stopper, and whatever the material the plunger stopper may be made of.

Moreover, plunger rods are usually made of plastic. Plunger rods are generally single used and discarded with the prefilled injection device once the injection is completed. Plastic plunger rods contribute to the global plastic waste generated by the sector of medical devices.

There is therefore the further need for a plunger rod that, not only would be capable of realizing a safe connection with any plunger stopper of an injection device whatever the design and/or material of such a plunger stopper, but that in addition would be removable from said plunger stopper and would be reusable with the plunger stopper of another prefilled injection device, as many times as possible.

A first aspect of the invention is a plunger rod intended to be connected to a proximal cavity of a plunger stopper of an injection device, said plunger rod comprising :
- A rod having a proximal end and a distal end,
- A sleeve configured to receive at least the distal end of the rod, a distal portion of said sleeve being provided with at least one movable part capable of moving from a first radial position to a second radial position under the effect of a force applied on said movable part,
- Said rod being capable of sliding in translation within said sleeve from a first axial position, in which the distal end of said rod is not in contact with said movable part, said movable part is in its first radial position and the distal portion of said sleeve may freely enter in, and exit from, said proximal cavity of said plunger stopper, to a second axial position, in which the distal end of said rod has applied a force on said movable part to move said part in its second radial position, and said movable part is capable of harpooning an inner wall of said proximal cavity of said plunger stopper to connect said plunger rod to said plunger stopper, said rod being configured to translate from its first axial position to its second axial position under the effect of a distal pressure exerted on its proximal end,
- Releasable locking means configured for temporary locking said rod in its second axial position.

In embodiments, the plunger rod may further comprise biasing means configured for automatically moving said rod from its second axial position to its first axial position when said releasable locking means are released.

The plunger rod of the invention may be connected to the proximal cavity of a plunger stopper whatever the design of the inner wall of said cavity. In particular, the inner wall of the proximal cavity may be provided with a thread of any dimensions or design, the movable part of the plunger rod of the invention will harpoon the inner wall of the cavity when moving from its first radial position to its second radial position, and will therefore attach the plunger rod to the plunger stopper by means of interference fit or by hooking, and not by means of screwing. The plunger rod of the invention is therefore not screwed into the plunger stopper, but it is fixed into the plunger stopper by means of the movable part harpooning the inner wall of the cavity. Indeed, plunger stoppers are usually made of elastomeric material, such as rubber, and the movable part can easily harpoon a wall made of such a material.

The plunger rod of the invention therefore allows simplifying the connecting step between the plunger rod and the plunger stopper. In particular, thanks to the plunger rod of the invention, the end-user no longer needs to screw the plunger rod into the plunger stopper. The risk that the plunger rod bend and/or break during the connecting step with the plunger stopper is in consequence significantly reduced. The plunger rod of the invention further allows removing one processing step in the case where the plunger rod is assembled with high speed machine at customer site.

In addition, thanks to the plunger rod of the invention, it is possible to use a single plunger rod for injection devices having plunger stoppers made of different materials and/or provided with connecting means, such as inner threads, having different designs. The plunger rod of the invention therefore helps streamlining the packaging, transportation and storage process of prefilled injection devices, as well as the use of such prefilled injection devices by the end-user within healthcare premises such as hospitals.

In addition, the plunger rod of the invention may easily be disconnected from a plunger stopper to which it has been attached as described above. Thanks to the biasing means of the plunger rod of the invention, the movable part may be easily detached from the inner wall of the cavity in which it is fixed. The movable part is allowed to move back to its first radial position, where the rod may be easily removed from the proximal cavity of the plunger stopper. Therefore, the end-user does not need to perform an unscrewing step in order to disconnect the plunger rod from the plunger stopper. Thanks to the plunger rod of the invention, the disconnection step is rendered very simple.

The plunger rod of the invention therefore further helps reducing the global plastic waste generated by the medical sector and is therefore environment friendly. Indeed, thanks to the plunger rod of the invention, it is possible to reuse the same plunger rod for a plurality of prefilled injection devices, thereby saving a significant amount of plunger rods and avoiding unnecessary plastic waste.

In embodiments, the proximal end of said rod is being provided with a button cap, and a proximal portion of the sleeve is being provided with a radial flange, and wherein said biasing means comprise a coil spring abutting on both a proximal surface of said radial flange and on a distal surface of said cap, said coil spring being capable of transitioning from a rest position where said rod is in its first axial position, to a stressed state where said rod is in its second axial position. Such embodiments allow performing the connection of the plunger rod to the plunger stopper in a simple manner, as the end user simply has to push distally on the cap of the plunger rod to realize the connection.

In embodiments, said rod being configured to rotate with respect to said sleeve, said releasable locking means comprise at least one hook extending distally from said cap and at least one partial circumferential groove located on said radial flange, said hook and said partial circumferential groove being configured to :
- engage one another to lock said rod in its second axial position when one exerts a distal pressure on said cap so as to put said coil spring in its stressed state, and further rotates said rod with respect to said sleeve in a first direction, and
- disengage from one another to free said rod from its second axial position and move it automatically to its first axial position, when one rotates said rod with respect to said sleeve contrary to said first direction and releases said distal pressure exerted on said cap.

Such embodiments allow locking the plunger rod in its connected configuration with the plunger stopper in a simple manner. The end user can therefore feel confident that the connection between the plunger rod and the plunger stopper is efficient and secure and that the injection step may be performed safely. Such embodiments further allow unlocking the plunger rod from the plunger stopper in a very simple manner.

In embodiments, the distal end of said rod is provided with an extension part configured to exert a radial force on said movable part to move said movable part from its second radial position to its first radial position when said rod moves back from its second axial position to its first axial position. Such an extension part may be located distally from said movable part and may be provided with inclined surfaces capable of cooperating with complementary inclined surfaces located on said movable part, in order to move the movable part in the centripetal direction when the extension part is moved in the proximal direction as the rod moves back from its second axial position to its first axial position.

The distal portion of the sleeve may be provided with more than one movable part; for example, the distal portion of the sleeve may be provided with two, three or four movable parts, that may be regularly distributed on a circumference of the sleeve. In embodiments, the distal portion of the sleeve is provided with two diametrically opposed movable parts. Such embodiments allow obtaining a secured and stable connection between the plunger rod and the plunger stopper, as two movable parts are anchored in the inner wall of the proximal cavity of the plunger stopper.

In embodiments, the plunger rod may further comprise a sheath dimensioned to surround said sleeve excepted said movable part, said sheath having an outer diameter substantially equal to the outer diameter of the distal portion of said sleeve when said movable part is in its first radial position. The sheath is preferably fixed in translation with respect to the sleeve. In such embodiments, the outer surface of the distal portion of the sleeve is flush with the outer surface of the sheath when the movable part(s) is/are in their first radial position : as such, the movable part(s) do not project outwardly radially from the sheath and the sleeve is therefore protected. Such embodiments are particularly convenient and user friendly, as the end user needs not taking care not to touch and/or damage the sleeve when handling the plunger rod.

In embodiments where the plunger rod comprises a sheath, the proximal end of said rod may be provided with a cap, and a proximal portion of the sheath may be provided with a radial flange, said biasing means may comprise a coil spring bearing on one hand on a proximal surface of said radial flange and on the other hand on a distal surface of said cap, said coil spring being capable of transitioning from a rest state where said rod is in its first axial position, to a stressed state where said rod is in its second axial position.

In such embodiments, the rod may be configured to rotate with respect to said sheath, said releasable locking means may comprise at least one hook extending distally from said cap and at least one partial circumferential groove located on said radial flange, said hook and said partial circumferential groove being configured to :
- engage one another to lock said rod in its second axial position when one exerts a distal pressure on said cap so as to put said coil spring in its stressed state, and further rotates said rod with respect to said sheath in a first direction, and
- disengage from one another to free said rod from its second axial position and move it automatically to its first axial position, when one rotates said rod with respect to said sheath contrary to said first direction and releases said distal pressure exerted on said cap.

In embodiments, said distal portion of said sleeve comprises at least one outwardly radially flexible leg, a distal end of said outwardly radially flexible leg forming said movable part. Alternatively, the distal portion of the sleeve may comprise two diametrically opposed outwardly radially flexible legs, a distal end of each outwardly radially flexible leg forming a movable part. Alternatively, the distal portion of the sleeve may comprise three outwardly radially flexible legs regularly distributed circumferentially, a distal end of each outwardly radially flexible leg forming a movable part. In such embodiments, the movable part may for example be in its first radial position when the flexible leg is in its rest state and the movable part may be in its second radial position when the flexible leg has been moved in the outwardly radially direction under the pressure applied by the distal end of the rod. When the distal end of the rod moves back to its first axial position, the flexible leg goes naturally back to its rest state, thereby causing the movable part to move automatically from its second radial position to its first radial position. Such embodiments are therefore very easy to manufacture and may be easily industrially implemented.

In embodiments, the movable part comprises an outer radial projection. Such outer radial projection is preferably configured to anchor in the inner wall of the proximal cavity under the centrifugal pressure exerted on the movable part by the distal end of the rod.

Another aspect of the invention is a method for connecting a plunger rod according as described above to the proximal cavity of a plunger stopper of an injection device, said method comprising the following steps :
- providing the plunger rod with the rod in its first axial position,
- inserting the distal portion of the sleeve inside the proximal cavity of the plunger stopper,
- applying a distal pressure onto the proximal end of the rod to guide the rod in its second axial position,
- activating the locking means to temporary lock the rod in its second axial position.

Another aspect of the invention is a method for disconnecting a plunger rod as described above which has been connected to the proximal cavity of a plunger stopper according to the connecting method above, comprising the following steps :
- deactivating the locking means,
- releasing all distal pressure applied on the proximal end of said rod to put said rod in its first axial position,
- removing the distal portion of the sleeve from the proximal cavity of the plunger stopper.

The invention and the advantages arising therefrom will clearly emerge from the detailed description that is given below with reference to the appended drawings as follows :
- Figure 1 is a perspective view of a first embodiment of the plunger rod of the invention inserted into the plunger stopper of an injection device, before connection,
- Figure 2 is a cross section view of the plunger stopper and plunger rod of Figure 1,
- Figure 3 is a detailed view of the plunger stopper and of the distal portion of the sleeve of the plunger rod of Figure 2,
- Figure 4 is a detailed view of the proximal region of the plunger rod of Figure 2,
- Figure 5 is a detailed perspective view of the distal portion of the sleeve of the plunger rod of Figure 2,
- Figure 6 is a perspective view of the plunger stopper and plunger rod of Figure 1, once the plunger rod is connected to the plunger stopper,
- Figure 7 is a cross section view of the plunger stopper and plunger rod of Figure 6,
- Figure 8 is a detailed view of the plunger stopper and of the distal portion of the sleeve of the plunger rod of Figure 6,
- Figure 9 is a detailed view of the proximal region of the plunger rod of Figure 6,
- Figure 10 is a detailed view showing the locking means locking of the plunger rod of Figure 6,
- Figure 11 is a cross section view of a second embodiment of the plunger rod of the invention inserted into a plunger stopper, before connection,
- Figure 12 is a detailed view of the plunger stopper and of the distal portion of the sleeve of the plunger rod of Figure 11,
- Figure 13 is a perspective view of the plunger stopper and of the distal portion of the sleeve of the plunger rod of Figure 12,
- Figure 14 is a cross section view of the plunger rod of Figure 11 once connected into the plunger stopper,
- Figure 15 is a detailed view of view of the plunger stopper and of the distal portion of the sleeve of the plunger rod of Figure 14,
- Figure 16 is a perspective view of the plunger stopper and of the distal portion of the sleeve of the plunger rod of Figure 15,
- Figure 17 is a cross section view of a third embodiment of the plunger rod of the invention inserted into a plunger stopper, before connection,
- Figure 18 is a detailed view of the proximal region of the plunger rod of Figure 17,
- Figure 19 is a perspective view of the distal portion of the sleeve and of the distal region of the sheath of the plunger rod of Figure 17,
- Figure 20 is a detailed view of the distal portion of the sleeve and of the distal region of the sheath of the plunger rod of Figure 17,
- Figure 21 is a cross section view of the plunger rod of Figure 17 once connected into the plunger stopper,
- Figure 22 is a detailed view of the proximal region of the plunger rod of Figure 17,
- Figure 23 is a detailed view showing the locking means of the plunger rod of Figure 17,
- Figure 24 is a detailed view of the distal portion of the sleeve and of the distal region of the sheath of the plunger rod of Figure 21,
- Figure 25 is a perspective view of the distal portion of the sleeve and of the distal region of the sheath of the plunger rod of Figure 21.

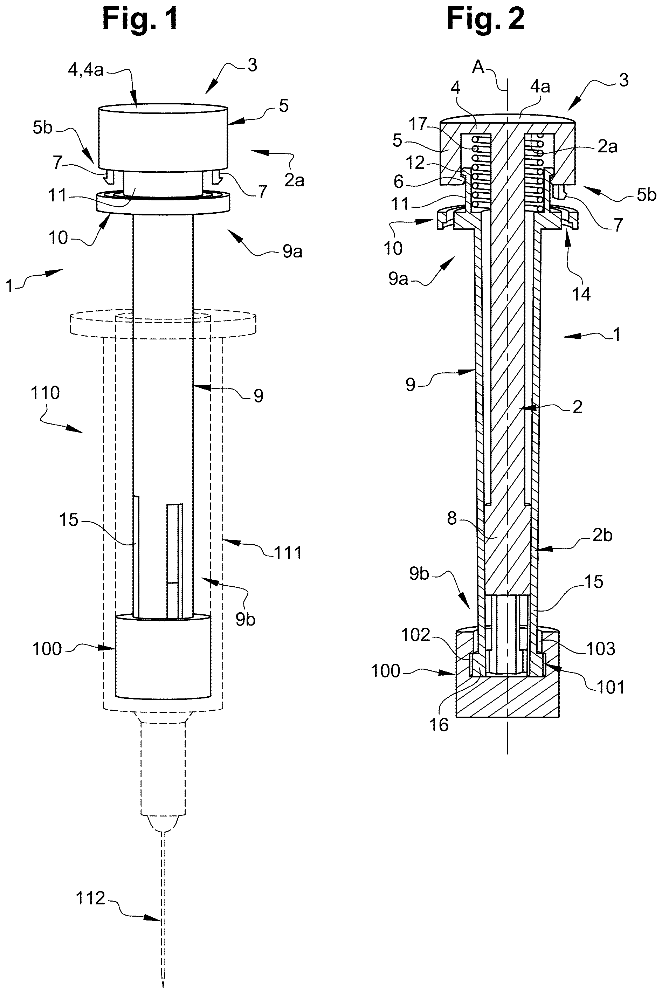

With reference to Figure 1 is shown a plunger rod 1 of the invention intended to be connected to a plunger stopper 100. The plunger stopper 100 is intended to be inserted into the container 111 of an injection device 110 shown in dotted line in Figure 1. The plunger stopper 100 is usually made of an elastomeric material, like rubber, and is used for sealing the proximal end of the container 111.

When the container 111 of the injection device contains a product to be injected, the plunger stopper 100 may be pushed distally in order to expel the product from the injection device 110 through the needle 112. When the container 111 is empty, as shown in Figure 1, the plunger stopper 100 may be moved in the proximal direction to cause the needle 112 to suck a product stored in a separate vial into the container 111.

The plunger rod 1 is intended to be connected to the plunger stopper 100 in a secured manner in order to allow an end user to confidently move the plunger stopper 100 in the distal direction and/or in the proximal direction, depending on the intended use.

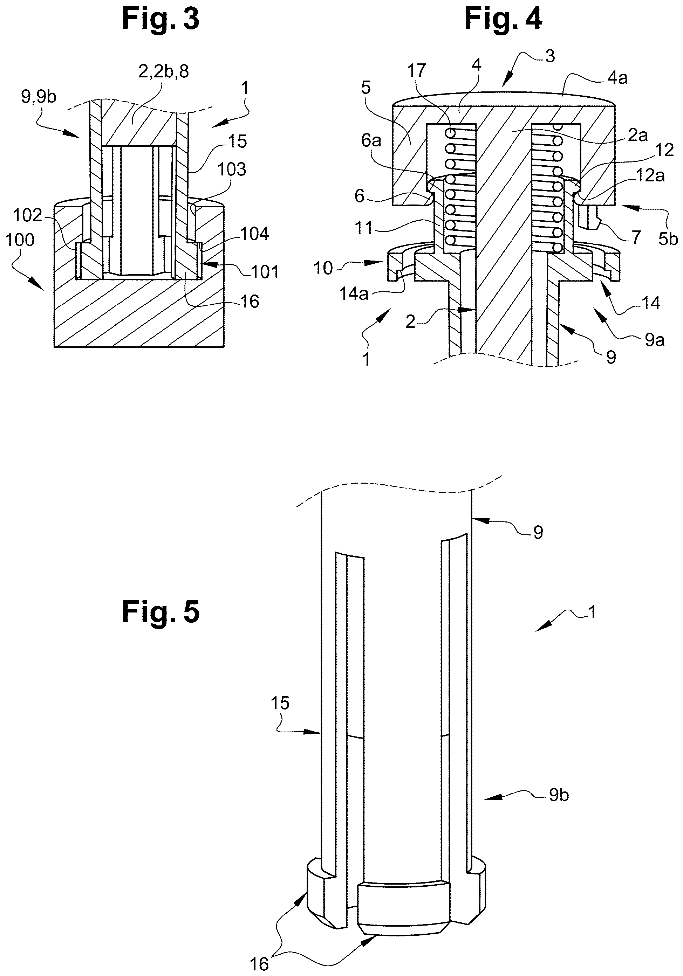

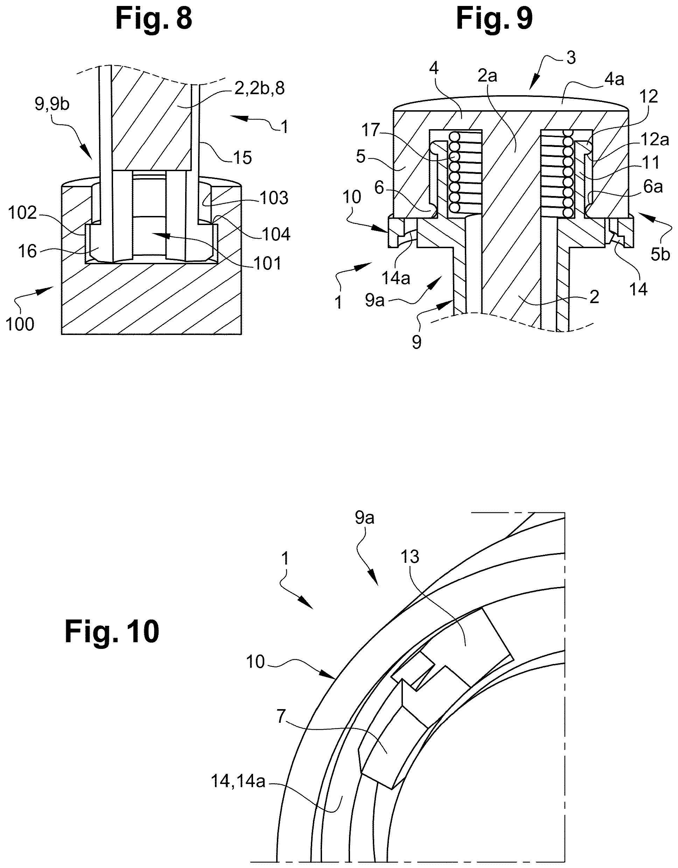

With reference to Figures 3 and 8, the plunger stopper 100 is provided with a proximal open cavity 101. Although not shown in the figures, the inner wall 102 of this proximal cavity may be provided with an inner thread, as is usually the case with commercially available plunger stoppers. In the example shown, the inner wall 102 of the proximal cavity 101 is provided with a proximal inner rib 103 forming an abutment surface 104.

With reference to Figures 1-10, the plunger rod 1 has a longitudinal axis A and comprises a rod 2 aligned on said axis. The rod 2 has a proximal end 2a and a distal end 2b. In the example shown, the proximal end 2a of the rod is provided with a cap 3 formed of a transversal wall 4 from which a tubular wall 5 extends in the distal direction. As will appear later in the description, the proximal face of the transversal wall 4 may form a pushing surface 4a for the end user to move the plunger rod 2 in the distal direction.

The tubular wall 5 is provided at its distal end 5b with an inner rim 6 forming a proximal abutment surface 6a. The distal end 5b of the tubular wall 5 is further provided with two (see Figure 1) diametrically opposed distal hooks 7, the function of which will be explained later on.

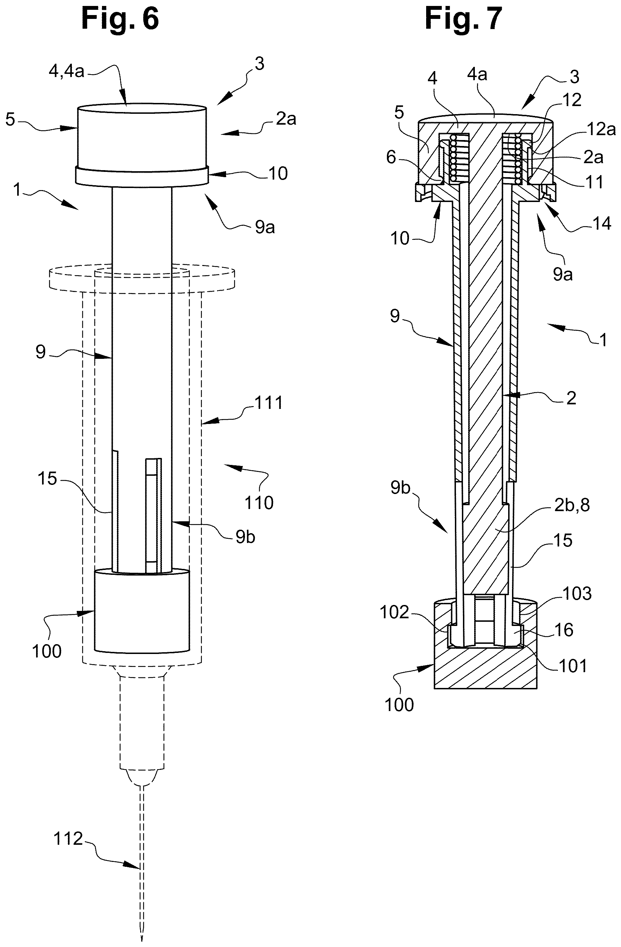

In the example shown, the distal end 2b of the rod 2 is provided with an end part 8 showing an outer diameter of larger dimension than the outer diameter of the rest of the rod 2. In embodiments not shown, the distal end 2b of the rod 2 could show the same outer dimensions as the rest of the rod, as long as, as will appear below, the outer dimensions of the distal end 2b of the rod 2 allow moving the outer projections 16 of the flexible legs 15 of the sleeve 9 (see Figure 7) in the centrifugal direction.

The plunger rod 2 further comprises a sleeve 9 having a proximal portion 9a and a distal portion 9b. The sleeve 9 is shaped and dimensioned to receive a distal region of the rod 2, and at least the distal end 2b of said rod 2. The proximal portion 9a of the sleeve 9 is provided with a radial flange 10 from which a tubular wall 11 extends in a proximal direction. The proximal end of the tubular wall 11 is provided with an outer rim 12 forming a distal abutment surface 12a.

The radial flange 10 is provided with two (only one being visible on Figure 10) diametrically opposed through holes 13 dimensioned to allow the passage there through of the distal hooks 7, and with two adjacent partial circumferential grooves 14 forming distal abutment surfaces 14a.

In the example shown, the distal portion 9b of the sleeve 9 is provided with three flexible legs 15 capable of deflecting in the outward radial direction under the effect of a centrifugal force exerted thereon. The flexible legs 15 are regularly distributed along a circumference of the sleeve 9. In embodiments not shown, the number of flexible legs 15 could be less than three, such as one or two, or alternatively more than two, like for example four. The distal end of each flexible leg 15 is provided with an outer projection 16. Each outer projection 16 therefore forms a movable part capable of moving from a first radial position to a second radial position when a centrifugal force is applied on the flexible legs 15.

The plunger rod 2 is further provided with a coil spring 17 lodged between the radial flange 10 and the transversal wall 4 of the cap 3 : the proximal end of the coil spring 17 bears on the distal face of the transversal wall 4 and the distal end of the coil spring 17 bears on the proximal face of the radial flange 10.

As will appear from the description below, the rod 2 is received in translation within the sleeve 9 and is capable of translating from a first axial position, as shown in Figures 1-5 to a second axial position, distally spaced from said first axial position, as shown in Figures 6-10, under the effect of a distal pressure exerted on the pushing surface 4a by an end user.

The connection of the plunger rod 1 to the plunger stopper 100 will now be described in reference to Figures 1-10.

With reference to Figures 1-5, the plunger rod 1 is not connected to the plunger stopper 100 and the rod 2 is in its first axial position. In such a position, the coil spring 17 is in a rest state, the distal end of the tubular wall 5 of the cap 3 is not in contact with the proximal face of the radial flange 10, and the coil spring 17 couples the rod 2 to the sleeve 9 by means of the distal abutment surface 12a of the outer rim 12 of the tubular wall 11 of the radial flange 10 resting on the proximal abutment surface 6a of the inner rim 6 of the tubular wall 5 of the cap 3 (see Figure 4). In this position, the distal end 2b of the rod 2 is not in contact with the distal ends of the flexible legs 15 and these flexible legs 15 are in a rest state. As a consequence, the outer projections 16 are in their first radial position. As a result, the outer dimensions of the distal portion 9b of the sleeve 9 are smaller than the inner dimensions of the inner wall 102 of the proximal open cavity 101 of the plunger stopper 100. The distal portion 9b of the sleeve 9 is therefore easily inserted in the proximal open cavity 101 of the plunger stopper 100, as can be seen in Figures 2 and 3.

Once the distal portion 9b of the sleeve 9 is inserted in the proximal open cavity 101 of the plunger stopper 100, the end user who wishes to connect the plunger rod 1 to the plunger stopper 100 simply applies a distal pressure onto the pushing surface 4a of the transversal wall 4 of the cap 3 of the plunger rod 2. Under this distal pressure, the distal end of the tubular wall 5 of the cap 3 comes in contact with the proximal face of the radial flange 10 and the coil spring 17 transitions to its stressed state. The distal movement of the rod 2 has caused the rod 2 to move from its first axial position to its second axial position, as shown in Figures 6-10. The end part 8 of the distal end 2b of the rod 2 has come in contact with the distal ends of the flexible legs 15 and has applied a centrifugal force onto the distal ends of these flexible legs 15, causing these flexible legs 15 to deflect outwardly radially. This has caused the outer projections 16 to move from their first radial position to their second radial position, as shown in Figure 8. In this second radial position, each outer projection 16 harpoons the inner wall 102 of the proximal open cavity 101 of the plunger stopper 100, thereby realizing a safe connection of the plunger rod 2 to the plunger stopper 100. Thanks to the centrifugal pressure exerted by the end part 8 onto the outer projections 16, the outer projections 16 anchor into the inner wall 102 of the proximal open cavity 101, regardless of the design of this inner wall 102, and in particular, independently from any inner thread that may be present on said inner wall 102. The efficiency of the connection may be reinforced by the presence of the abutment surface 104 of the inner rim 103 of the plunger stopper 100, said abutment surface 104 preventing the outer projections 16 to move in the proximal direction.

When the distal end of the tubular wall 5 of the cap 3 has come in contact with the proximal face of the radial flange 10, each distal hook 7 has passed through the corresponding through hole 13. As a consequence, in order to lock the rod 2 in its second axial position, the end user needs only to rotate the cap 3 with respect to the sleeve 9 in a first direction in order to bring the distal hooks 7 in contact with the abutment surfaces 14a of the partial cirfumferential grooves 14, as shown in Figure 10. The coil spring 17 in its stressed state and the distal hooks 7 engaged in the abutment surfaces 14a of the partial cirfumferential grooves 14 form releasable locking means of the rod 2 in its second axial position. The end user can feel confident that the connection between the plunger rod 1 and the plunger stopper 100 is efficient and secured and he can manipulate the plunger rod 1 safely in order to move the plunger stopper 100 within the container 111 of the injection device 110.

When the end user wishes to disconnect the plunger rod 1 from the plunger stopper 100, he simply needs to rotate the cap 3 with respect to the sleeve 9 contrary to the first direction previously used in order to disengage the distal hooks 7 from the partial circumferential grooves 14. The end user then releases the distal pressure he exerts on the pushing surface 4a of the cap 3 and the rod 2 comes automatically back to its first axial position, under the effect of the coil spring 17 naturally transitioning to its rest state, as shown in Figure 2. During this operation, the end part 8 of the distal end 2b of the rod 2 has left the distal ends of the flexible legs 15 which have come back to their rest state. The outer projections 16 have therefore moved from their second radial position to their first radial position, as shown in Figure 3, and the plunger rod 1 may easily be disconnected from the plunger stopper 100 by simply removing the distal portion 9b of the sleeve 9 from the proximal open cavity 101 of the plunger stopper 100.

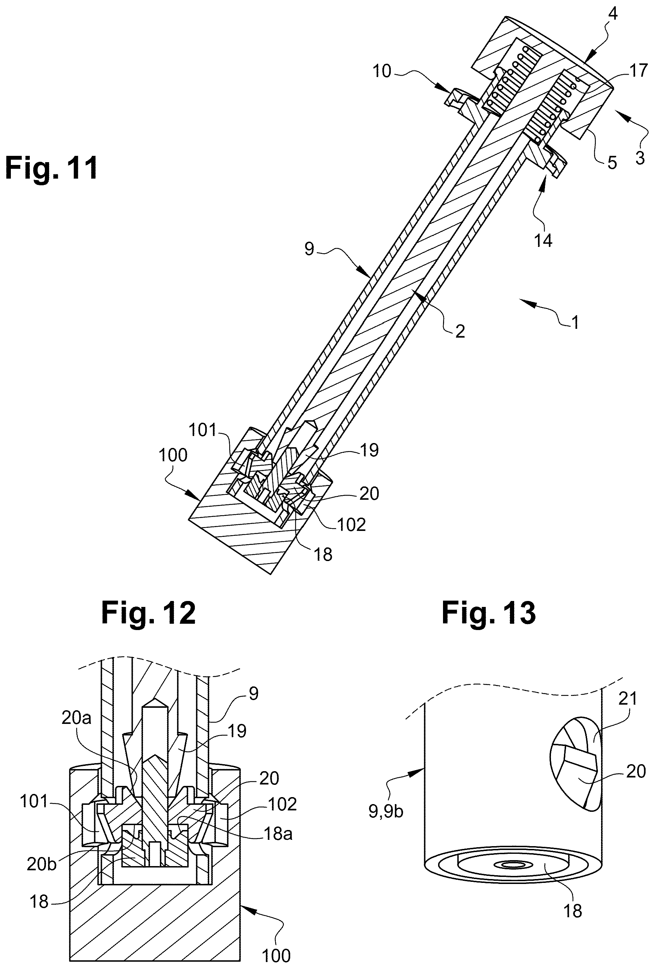

Figures 11-16 relate to a second embodiment of the plunger rod 1 of the invention, in which the flexible legs and the outer projections are replaced by non flexible movable parts; The elements which are identical to that of the plunger rod 1 of Figures 1-10 are designated by the same references.

With reference to these figures, the distal end of the rod 2 is provided with an extension part 18 showing a hollow conical surface 18a extending in the proximal direction. The distal region of the rod 2 further comprises an outer conical part 19, tapering in the distal direction, said outer conical part 19 being located proximal to the extension part 18. The sleeve 9 receives in its distal portion 9b two diametrically opposed mobile parts 20, each mobile part 20 comprising a proximal inclined surface 20a and a distal inclined surface 20b. The wall of the sleeve 9 is provided with two diametrically opposed windows 21, each window 21 facing a mobile part 20. The mobile parts 20 and the windows 21 of the sleeve 9 are axially located between the extension part 18 and the outer conical part 19 of the rod 2.

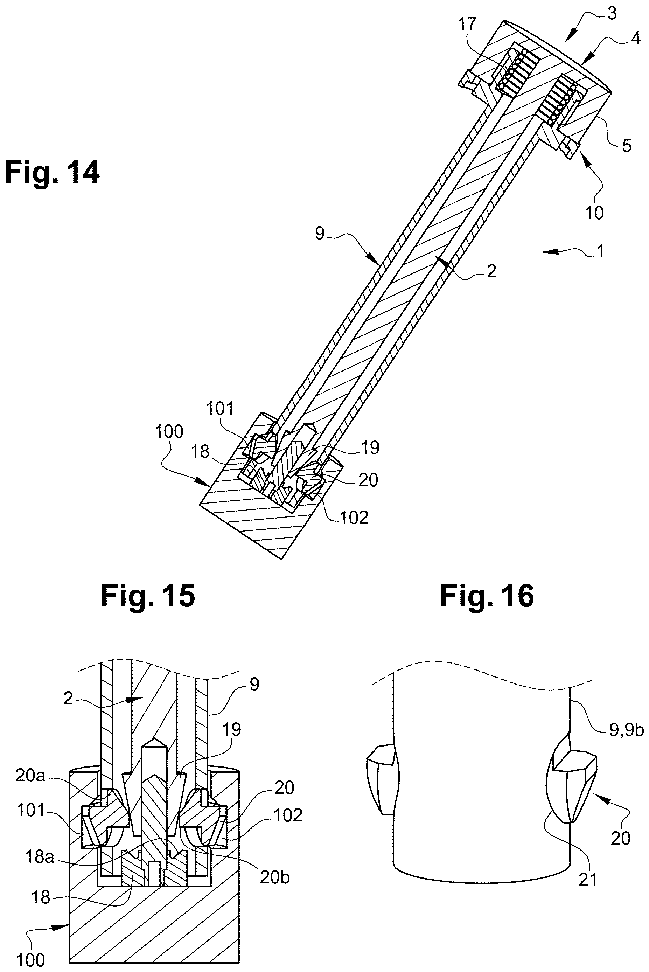

The rod 2 is shown in its first axial position in Figures 11-13, and in its second axial position in Figures 14-16.

With reference to Figures 11-13, the outer conical part 19 of the rod 2 is not in contact with the mobile parts 20, which are entirely located within the walls of the sleeve 9. The outer dimensions of the distal portion 9b of the sleeve 9 are less than the inner dimensions of the proximal open cavity 101 of the plunger stopper 100.

When an end user wishes to connect the plunger rod 1 to the plunger stopper 100, he applies a pressure onto the transversal wall 4 of the cap 3 of the plunger rod 1 as described above. When the rod 2 moves to its second axial position, as shown in Figures 14-16, the outer conical part 19 comes in contact with the proximal inclined surfaces 20a of the mobile parts 20 and applies a centrifugal force on these surfaces, thereby causing the mobile parts 20 to move in the centrifugal direction. The mobile parts 20 therefore move from their first radial position, as shown in Figures 12 and 13, to their second radial position, as shown in Figure 15 and 16. The mobile parts 20 therefore projects out from the windows 21 of the wall of the sleeve 9 and they harpoon the inner wall 102 of the proximal open cavity 101 of the plunger stopper, thereby realizing the connection of the plunger rod 1 to the plunger stopper 100.

In this position, the end user may lock the rod 2 in its second axial position in the same manner as described above with reference to Figures 1-10.

When the end user wishes to disconnect the plunger rod 1 from the plunger stopper 100, he releases the coil spring 17 as described above in Figures 1-10.

When the rod 2 moves proximally from its second axial position to its first axial position, the extension part 18 moves equally proximally and its hollow conical surface 18a comes in contact with the distal inclined surfaces 20b of the mobile parts 20 (see Figure 15). The hollow conical surface 18a slides onto the distal inclined surfaces 20b of the mobile parts 20, thereby applying a centripetal force on theses surfaces and causing the mobile parts 20 to move backwards in the direction of their first radial position. As a consequence, the mobile parts 20 withdraw within the interior of the sleeve 9, as shown in Figure 13. The end user may therefore easily disconnect the plunger rod 1 from the plunger stopper 100 by removing the distal portion 9b of the sleeve 9 from the proximal open cavity 101 of the plunger stopper 100.

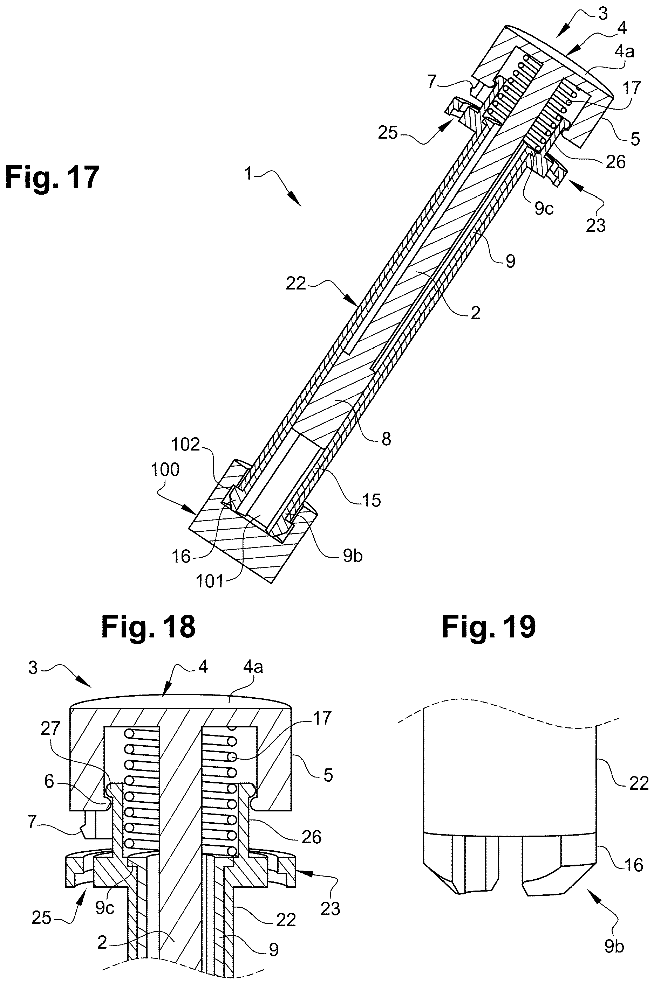

Figures 17-25 relate to a third embodiment of the plunger rod 1 of the invention, similar to that of Figures 1-10, but additionally comprising an outer sheath surrounding the sleeve. The elements which are identical to that of the plunger rod 1 of Figures 1-10 are designated by the same references.

With reference to these figures, the plunger rod 1 comprises a rod 2 identical to that of Figures 1-10, comprising a cap 3 and an end part 8. The plunger rod 1 further comprises a sleeve 9, the distal portion 9b of which is provided with two diametrically opposed flexible legs 15. The distal end of each flexible leg 15 is provided with an outer projection 16. Contrary to the embodiment of Figures 1-10, the proximal end of the sleeve 9 is free of any radial flange and comprises an outer rim 9c.

The plunger rod 1 of Figures 17-25 further comprises an outer sheath 22 surrounding the sleeve 9, except for the outer projections 16. The proximal end of the outer sheath 22 is provided with a radial flange 23, to which is fixed the outer rim 9c of the sleeve 9. The sleeve 9 and the outer sheath 22 are fixed to each other. With reference to Figure 19, the outer sheath 22 has an outer diameter substantially equal to the outer diameter of the distal portion 9b of the sleeve 9 when the outer projections 16 are in their first radial position.

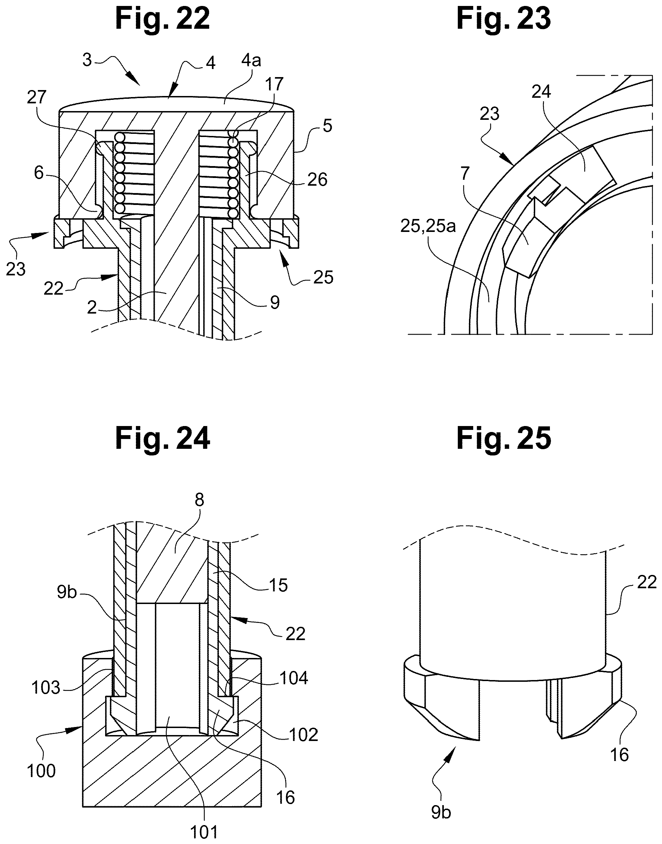

The radial flange 23 is similar to the radial flange 10 of embodiments of Figures 1-10, except that it is provided at the proximal end of the outer sheath 22 rather than at the proximal end of the sleeve 9. As a consequence, the radial flange 23 is provided with two (only one being visible on Figure 23) diametrically opposed through holes 24 dimensioned to allow the passage there through of the distal hooks 7, and with two adjacent partial circumferential grooves 25 forming distal abutment surfaces 25a. The radial flange 23 is further provided with a tubular wall 26 extending in the proximal direction. The proximal end of the tubular wall 26 is provided with an outer rim 27 intended to cooperate with the inner rim 6 of the cap 3 in order to maintain the coil spring 17 in its rest state (see Figure 18).

The method for connecting the plunger rod 1 of Figures 17-25 is the same as that described above for the embodiment of Figures 1-10. The plunger rod 1 of Figures 17-25 has the advantage that the sleeve 9 is protected by the outer sheath 22 during the connecting and disconnecting operations. In particular, as visible on Figure 19, the outer surface of the outer projections 16 is flush with the outer surface of the outer sheath 22. This is very convenient for the end user who needs not taking care not to touch the sleeve and the outer projections when he manipulates the plunger rod 1.

With reference to Figures 17-20, the plunger rod 1 is not connected to the plunger stopper 100 and the rod 2 is in its first axial position. In such a position, the coil spring 17 is in a rest state, the distal end of the tubular wall 5 of the cap 3 is not in contact with the proximal face of the radial flange 23, and the coil spring 17 couples the rod 2 to the outer sheath 22 by means of the outer rim 27 of the tubular wall 26 of the radial flange 23 resting on the inner rim 6 of the tubular wall 5 of the cap 3 (see Figure 18). In this position, the distal end 2b of the rod 2 is not in contact with the distal ends of the flexible legs 15 and these flexible legs 15 are in a rest state. As a consequence, the outer projections 16 are in their first radial position, as shown in Figure 19. The outer dimensions of the distal portion 9b of the sleeve 9 and of the distal portion of the outer sheath 22 are smaller than the inner dimensions of the inner wall 102 of the proximal open cavity 101 of the plunger stopper 100. The distal portion 9b of the sleeve 9 is therefore easily inserted in the proximal open cavity 101 of the plunger stopper 100, as can be seen in Figure 17.

As previously described, when the end user then applies a distal pressure on the cap 3, the coil spring 17 transitions to its stressed state, and the end part 8 of the distal end 2b of the rod 2 comes in contact with the distal ends of the flexible legs 15 and applies a centrifugal force onto the distal ends of these flexible legs 15, causing these flexible legs 15 to deflect outwardly radially. The outer projections 16 to move from their first radial position to their second radial position, where they project out of the outer sheath 22, as shown in Figure 25. Thanks to the centrifugal pressure exerted by the end part 8 onto the outer projections 16, the outer projections 16 anchor into the inner wall 102 of the proximal open cavity 101, as shown in Figure 24, regardless of the design of this inner wall 102, and in particular, independently from any inner thread that may be present on said inner wall 102. The efficiency of the connection may be reinforced by the presence of the abutment surface 104 of the inner rim 103 of the plunger stopper 100, said abutment surface 104 preventing the outer projections 16 to move in the proximal direction.

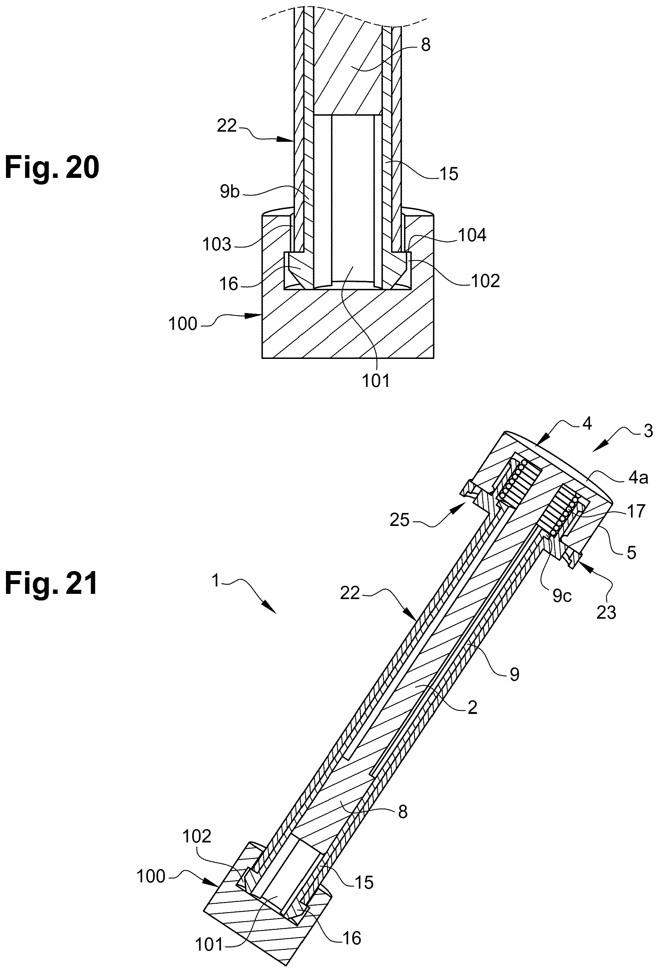

The locking of the rod 2 in this second axial position (shown in Figures 21-25) may be performed similarly as previously described for the embodiment of Figures 1-10. To do so, the end user needs only to rotate the cap 3 with respect to the outer sheath 22 in a first direction in order to bring the distal hooks 7 in contact with the abutment surfaces 25a of the partial cirfumferential grooves 25, as shown in Figure 25. The end user can feel confident that the connection between the plunger rod 1 and the plunger stopper 100 is efficient and secured and that he can manipulate the plunger rod 1 safely in order to move the plunger stopper 100 within the container 111 of the injection device 110.

In order to disconnect the plunger rod 1 from the plunger stopper 100, the end user simply rotates the cap 3 with respect to the outer sheath 22 contrary to the first direction previously used in order to disengage the distal hooks 7 from the partial circumferential grooves 25. The end user then releases the distal pressure he exerts on the pushing surface 4a of the cap 3 and the rod 2 comes automatically back to its first axial position, under the effect of the coil spring 17 naturally transitioning to its rest state, as shown in Figure 17. During this step, the flexible legs 15 have come back to their rest state, and the outer projections 16 have come back to their first radial position, as shown in Figure 19. The end user can therefore easily disconnect the plunger rod 1 from the plunger stopper 100 by removing the distal portion of the outer sheath 22 from the proximal cavity 101 of the plunger stopper 100.

The plunger rod of the invention therefore allows simplifying the connecting step of a plunger rod to a plunger stopper as a screwing step is no longer necessary. In addition, the plunger rod of the invention may be connected safely to any plunger stopper, whatever the material of the plunger stopper and the design of the inner wall of the cavity of the plunger stopper. A single plunger rod may be used for a plurality of plunger stoppers and/or injections devices, thereby helping reducing the plastic waste generated in the sector of medical devices.

## Claims

1. Plunger rod (1) intended to be connected to a proximal cavity (101) of a plunger stopper (100) of an injection device (110), said plunger rod (1) comprising :
- A rod (2) having a proximal end (2a) and a distal end (2b),
- A sleeve (9) configured to receive at least the distal end (2b) of the rod (2), a distal portion (9b) of said sleeve (9) being provided with at least one movable part (15, 16) capable of moving from a first radial position to a second radial position under the effect of a force applied on said movable part (15, 16),
- Said rod (2) being capable of sliding in translation within said sleeve (9) from a first axial position, in which the distal end (2b, 8; 19) of said rod (2) is not in contact with said movable part (15, 16; 20), said movable part (15, 16; 20) is in its first radial position and the distal portion (9b) of said sleeve may freely enter in, and exit from, said proximal cavity (101) of said plunger stopper (100), to a second axial position, in which the distal end (2b, 8; 19) of said rod (2) has applied a force on said movable part (15, 16; 20) to move said part in its second radial position, and said movable part (15, 16; 20) is capable of harpooning an inner wall (102) of said proximal cavity (101) of said plunger stopper (100) to connect said plunger rod (1) to said plunger stopper (100), said rod (2) being configured to translate from its first axial position to its second axial position under the effect of a distal pressure exerted on its proximal end (2a, 3, 4, 4a),
**characterized in that** the plunger rod further comprises
- Releasable locking means (7, 14, 14a; 25, 25a) configured for temporary locking said rod (2) in its second axial position.

2. Plunger rod (1) according to claim 1, further comprising biasing means (17) configured for automatically moving said rod (2) from its second axial position to its first axial position when said releasable locking means (7, 14, 14a; 25, 25a) are released.

3. Plunger rod (1) according to claim 1 or 2, wherein the proximal end (2a) of said rod is being provided with a button cap (3), and a proximal portion (9a) of the sleeve (9) is being provided with a radial flange (10), and wherein said biasing means comprise a coil spring (17) abutting on both a proximal surface of said radial flange (10) and on a distal surface of said cap (3), said coil spring (17) being capable of transitioning from a rest position where said rod (2) is in its first axial position, to a stressed state where said rod (2) is in its second axial position.

4. Plunger rod (1) according to claim 3, wherein said rod (2) is being configured to rotate with respect to said sleeve (9), said releasable locking means comprising at least one hook (7) extending distally from said cap (3) and at least one partial circumferential groove (14) located on said radial flange (10), said hook (7) and said circumferential groove (14) being configured to :
- engage one another to lock said rod (2) in its second axial position when one exerts a distal pressure on said cap (3) so as to put said coil spring (17) in its stressed state, and further rotates said rod (2) with respect to said sleeve (9) in a first direction, and
- disengage from one another to free said rod (2) from its second axial position and move it automatically to its first axial position, when one rotates said rod (2) with respect to said sleeve (9) contrary to said first direction and releases said distal pressure exerted on said cap (3).

5. Plunger rod (1) according to any one of claims 1 to 4, wherein the distal portion (9b) of said sleeve (9) is provided with two diametrically opposed movable parts (20).

6. Plunger rod (1) according to any one of claims 1 to 5, wherein the distal end (2b) of said rod (2) is provided with an extension part (18) configured to exert a radial force on said movable part (20) to move said movable part (20) from its second radial position to its first radial position when said rod (2) moves back from its second axial position to its first axial position.

7. Plunger rod (1) according to claim 1 or 2, further comprising a sheath (22) dimensioned to surround said sleeve (9) excepted said movable part (16), said sheath (22) having an outer diameter substantially equal to the outer diameter of the distal portion (9b) of said sleeve (9) when said movable part (16) is in its first radial position.

8. Plunger rod (1) according to the preceding claim, wherein the proximal end (2a) of said rod (2) being provided with a cap (3), and a proximal portion of the sheath (22) being provided with a radial flange (23), said biasing means comprise a coil spring (17) bearing on one hand on a proximal surface of said radial flange (23) and on the other hand on a distal surface of said cap (3), said coil spring (17) being capable of transitioning from a rest state where said rod (2) is in its first axial position, to a stressed state where said rod (2) is in its second axial position.

9. Plunger rod (1) according to the preceding claim wherein said rod (2) being configured to rotate with respect to said sheath (22), said releasable locking means comprise at least one hook (7) extending distally from said cap (3) and at least one partial circumferential groove (25) located on said radial flange (23), said hook (7) and said partial circumferential groove (25) being configured to :
- engage one another to lock said rod (2) in its second axial position when one exerts a distal pressure on said cap (3) so as to put said coil spring (17)in its stressed state, and further rotates said rod (2) with respect to said sheath (22) in a first direction, and
- disengage from one another to free said rod (2) from its second axial position and move it automatically to its first axial position, when one rotates said rod (2) with respect to said sheath (22) contrary to said first direction and releases said distal pressure exerted on said cap (3).

10. Plunger rod (1) according to any one of claims 1 to 4 and 7 to 9, wherein said distal portion (9b) of said sleeve (9) comprises at least one outwardly radially flexible leg (15), a distal end (16) of said outwardly radially flexible leg (15) forming said movable part.

11. Plunger rod (1) according to claim 10, wherein said distal portion of said sleeve (9) comprises two diametrically opposed outwardly radially flexible legs (15), a distal end (16) of each outwardly radially flexible leg (15) forming a movable part.

12. Plunger rod (1) according to claim 10, wherein said distal portion (9b) of said sleeve (9) comprises three outwardly radially flexible legs (15) regularly distributed circumferentially, a distal end (16) of each outwardly radially flexible leg (15) forming a movable part.

13. Plunger rod (1) according to nay one of claims 1 to 12, wherein the movable part comprises an outer radial projection (16).

14. Method for connecting a plunger rod (1) according to any one of claims 1 to 13 to the proximal cavity (101) of a plunger stopper (100) of an injection device, said method comprising the following steps :
- providing the plunger rod (1) with the rod (2) in its first axial position,
- inserting the distal portion (9b) of the sleeve (9) inside the proximal cavity (101) of the plunger stopper (100),
- applying a distal pressure onto the proximal end (2a, 3, 4, 4a) of the rod (2) to guide the rod (2) in its second axial position,
- activating the locking means (7, 14, 14a; 25, 25a) to temporary lock the rod (2) in its second axial position.

15. Method for disconnecting a plunger rod (1) according to any one of claims 1 to 13 which has been connected to the proximal cavity (101) of a plunger stopper (100) according to the method of claim 13, comprising the following steps :
- deactivating the locking means (7, 14, 14a; 25, 25a),
- releasing all distal pressure applied on the proximal end (2a, 3, 4, 4a) of said rod (2) to put said rod (2) in its first axial position,
- removing the distal portion (9b) of the sleeve (9) from the proximal cavity (101) of the plunger stopper (100).

## Patentansprüche

1. Kolbenstange (1), welche dazu bestimmt ist, mit einem proximalen Hohlraum (101) eines Kolbenstoppers (100) einer Injektionsvorrichtung (110) verbunden zu werden, wobei die Kolbenstange (1) umfasst:
- einen Stab (2) mit einem proximalen Ende (2a) und einem distalen Ende (2b),
- eine Hülse (9), welche dazu konfiguriert ist, zumindest das distale Ende (2b) des Stabs (2) aufzunehmen, wobei ein distaler Abschnitt (9b) der Hülse (9) mit zumindest einem beweglichen Teil (15, 16) versehen ist, welcher in der Lage ist, sich unter der Wirkung einer auf den beweglichen Teil (15, 16) ausgeübten Kraft von einer ersten radialen Position in eine zweite radiale Position zu bewegen,
- wobei der Stab (2) in der Lage ist, in Translation innerhalb der Hülse (9) aus einer ersten axialen Position, in welcher das distale Ende (2b, 8; 19) des Stabs (2) nicht in Kontakt mit dem beweglichen Teil (15, 16; 20) ist, wobei der bewegliche Teil (15, 16; 20) in seiner ersten radialen Position ist und der distale Abschnitt (9b) der Hülse frei in den proximalen Hohlraum (101) des Kolbenstoppers (100) ein- und austreten kann, in eine zweite axiale Position zu gleiten, in welcher das distale Ende (2b, 8; 19) des Stabs (2) eine Kraft auf den beweglichen Teil (15, 16; 20) ausgeübt hat, um den beweglichen Teil in seine zweite radiale Position zu bewegen, und der bewegliche Teil (15, 16; 20) in der Lage ist, eine Innenwand (102) des proximalen Hohlraums (101) des Kolbenstoppers (100) zu harpunieren, um die Kolbenstange (1) mit dem Kolbenstopper (100) zu verbinden, wobei der Stab (2) dazu konfiguriert ist, unter der Wirkung eines distalen Drucks, welcher auf sein proximales Ende (2a, 3, 4, 4a) angewendet wird, von seiner ersten axialen Position in seine zweite axiale Position überzugehen,
**dadurch gekennzeichnet, dass** die Kolbenstange weiter umfasst:
- lösbare Verriegelungsmittel (7, 14, 14a; 25, 25a), welche dazu konfiguriert sind, den Stab (2) vorübergehend in seiner zweiten axialen Position zu verriegeln.

2. Kolbenstange (1) nach Anspruch 1, weiter umfassend Vorspannmittel (17) umfassend, welche dazu konfiguriert sind, den Stab (2) automatisch von seiner zweiten axialen Position in seine erste axiale Position zu bewegen, wenn die lösbaren Verriegelungsmittel (7, 14, 14a; 25, 25a) gelöst werden.

3. Kolbenstange (1) nach Anspruch 1 oder 2, wobei das proximale Ende (2a) des Stabs mit einer Knopfkappe (3) versehen ist, und ein proximaler Abschnitt (9a) der Hülse (9) mit einem radialen Flansch (10) versehen ist, und wobei die Vorspannmittel eine Schraubenfeder (17) umfassen, welche sowohl an eine proximale Fläche des radialen Flansches (10) als auch an eine distale Fläche der Kappe (3) anstößt, wobei die Schraubenfeder (17) in der Lage ist, aus einer Ruheposition, in welcher sich der Stab (2) in seiner ersten axialen Position befindet, in einen gespannten Zustand überzugehen, in welchem sich der Stab (2) in seiner zweiten axialen Position befindet.

4. Kolbenstange (1) nach Anspruch 3, wobei der Stab (2) dazu konfiguriert ist, sich in Bezug auf die Hülse (9) zu drehen, wobei die lösbaren Verriegelungsmittel zumindest einen Haken (7), welcher sich distal von der Kappe (3) erstreckt, und zumindest eine teilweise umlaufende Nut (14) umfassen, welche sich auf dem radialen Flansch (10) befindet, wobei der Haken (7) und die umlaufende Nut (14) dazu konfiguriert sind:
- ineinander einzugreifen, um den Stab (2) in seiner zweiten axialen Position zu verriegeln, wenn man einen distalen Druck auf die Kappe (3) anwendet, um die Schraubenfeder (17) in ihren gespannten Zustand zu versetzen, und weiter den Stab (2) in Bezug auf die Hülse (9) in eine erste Richtung zu drehen, und
- sich voneinander zu lösen, um den Stab (2) aus seiner zweiten axialen Position zu lösen und ihn automatisch in seine erste axiale Position zu bewegen, wenn man den Stab (2) in Bezug auf die Hülse (9) entgegen der ersten Richtung dreht und den auf die Kappe (3) angewendeten distalen Druck ablässt.

5. Kolbenstange (1) nach einem der Ansprüche 1 bis 4, wobei der distale Abschnitt (9b) der Hülse (9) mit zwei diametral gegenüberliegenden beweglichen Teilen (20) versehen ist.

6. Kolbenstange (1) nach einem der Ansprüche 1 bis 5, wobei das distale Ende (2b) des Stabs (2) mit einem Verlängerungsteil (18) versehen ist, welcher dazu konfiguriert ist, eine radiale Kraft auf den bewegliche Teil (20) anzuwenden, um den beweglichen Teil (20) von seiner zweiten radialen Position in seine erste radiale Position zu bewegen, wenn sich der Stab (2) von seiner zweiten axialen Position zurück in seine erste axiale Position bewegt.

7. Kolbenstange (1) nach Anspruch 1 oder 2, weiter einen Mantel (22) umfassend, welcher so dimensioniert ist, dass er die Hülse (9) mit Ausnahme des beweglichen Teils (16) umgibt, wobei der Mantel (22) einen Außendurchmesser aufweist, welcher im Wesentlichen gleich dem Außendurchmesser des distalen Abschnitts (9b) der Hülse (9) ist, wenn sich der bewegliche Teil (16) in seiner ersten radialen Position befindet.

8. Kolbenstange (1) nach dem vorstehenden Anspruch, wobei das proximale Ende (2a) des Stabs (2) mit einer Kappe (3) versehen ist, und ein proximaler Abschnitt der Mantels (22) mit einem radialen Flansch (23) versehen ist, wobei die Vorspannmittel eine Schraubenfeder (17) umfassen, welche einerseits auf einer proximalen Fläche des radialen Flansches (23) und andererseits auf einer distalen Fläche der Kappe (3) gelagert ist, wobei die Schraubenfeder (17) in der Lage ist, aus einem Ruhezustand, in welchem sich der Stab (2) in seiner ersten axialen Position befindet, in einen gespannten Zustand überzugehen, in welchem sich der Stab (2) in seinem zweiten axialen Position befindet.

9. Kolbenstange (1) nach dem vorstehenden Anspruch, wobei der Stab (2) dazu konfiguriert ist, sich in Bezug auf den Mantel (22) zu drehen, die lösbaren Verriegelungsmittel zumindest einen Haken (7), welcher sich distal von der Kappe (3) erstreckt, und zumindest eine teilweise umlaufende Nut (25) umfassen, welche sich auf dem radialen Flansch (23) befindet, wobei der Haken (7) und die teilweise umlaufende Nut (25) dazu konfiguriert sind:
- ineinander einzugreifen, um den Stab (2) in seiner zweiten axialen Position zu verriegeln, wenn man einen distalen Druck auf die Kappe (3) anwendet, um die Schraubenfeder (17) in ihren gespannten Zustand zu versetzen, und weiter den Stab (2) in Bezug auf den Mantel (22) in eine erste Richtung zu drehen, und
- sich voneinander zu lösen, um den Stab (2) aus seiner zweiten axialen Position zu lösen und ihn automatisch in seine erste axiale Position zu bewegen, wenn man den Stab (2) in Bezug auf den Mantel (22) entgegen der ersten Richtung dreht und den auf die Kappe (3) angewendeten distalen Druck ablässt.

10. Kolbenstange (1) nach einem der Ansprüche 1 bis 4 und 7 bis 9, wobei der distale Abschnitt (9b) der Hülse (9) zumindest einen nach außen radial flexiblen Schenkel (15) umfasst, wobei ein distales Ende (16) des nach außen radial flexiblen Schenkels (15) den beweglichen Teil bildet.

11. Kolbenstange (1) nach Anspruch 10, wobei der distale Abschnitt der Hülse (9) zwei diametral gegenüberliegende, nach außen radial flexible Schenkel (15) umfasst, wobei ein distales Ende (16) jedes nach außen radial flexiblen Schenkels (15) einen beweglichen Teil bildet.

12. Kolbenstange (1) nach Anspruch 10, wobei der distale Abschnitt (9b) der Hülse (9) drei nach außen radial flexible Schenkel (15) umfasst, welche umlaufend regelmäßig verteilt sind, wobei ein distales Ende (16) jedes nach außen radial flexiblen Schenkels (15) einen beweglichen Teil bildet.

13. Kolbenstange (1) nach einem der Ansprüche 1 bis 12, wobei der bewegliche Teil einen äußeren radialen Vorsprung (16) umfasst.

14. Verfahren zum Verbinden einer Kolbenstange (1) nach einem der Ansprüche 1 bis 13 mit dem proximalen Hohlraum (101) eines Kolbenstoppers (100) einer Injektionsvorrichtung, wobei das Verfahren die folgenden Schritte umfasst:
- Bereitstellen der Kolbenstange (1) mit dem Stab (2) in seiner ersten axialen Position,
- Einführen des distalen Abschnitts (9b) der Hülse (9) in den proximalen Hohlraum (101) des Kolbenstoppers (100),
- Anwenden eines distalen Drucks auf das proximale Ende (2a, 3, 4, 4a) des Stabs (2), um den Stab (2) in seine zweite axiale Position zu führen,
- Aktivieren der Verriegelungsmittel (7, 14, 14a; 25, 25a), um den Stab (2) vorübergehend in seiner zweiten axialen Position zu verriegeln.

15. Verfahren zum Trennen einer Kolbenstange (1) nach einem der Ansprüche 1 bis 13, welche mit dem proximalen Hohlraum (101) eines Kolbenstoppers (100) nach dem Verfahren von Anspruch 13 verbunden ist, umfassend die folgenden Schritte:
- Deaktivieren der Verriegelungsmittel (7, 14, 14a; 25, 25a),
- Lösen des gesamten distalen Drucks, welcher auf das proximale Ende (2a, 3, 4, 4a) des Stabs (2) ausgeübt wird, um den Stab (2) in seine erste axiale Position zu bringen,
- Entfernen des distalen Abschnitts (9b) der Hülse (9) aus dem proximalen Hohlraum (101) des Kolbenstoppers (100).

## Revendications

1. Tige de piston (1) destinée à être reliée à une cavité proximale (101) d'un bouchon de piston (100) d'un dispositif d'injection (110), ladite tige de piston (1) comprenant :
- une tige (2) ayant une extrémité proximale (2a) et une extrémité distale (2b),
- un manchon (9) configuré pour recevoir au moins l'extrémité distale (2b) de la tige (2), une portion distale (9b) dudit manchon (9) étant pourvue d'au moins une partie mobile (15, 16) capable de se déplacer d'une première position radiale à une seconde position radiale sous l'effet d'une force appliquée sur ladite partie mobile (15, 16),
- ladite tige (2) pouvant coulisser en translation à l'intérieur dudit manchon (9) depuis une première position axiale, dans laquelle l'extrémité distale (2b, 8 ; 19) de ladite tige (2) n'est pas en contact avec ladite partie mobile (15, 16 ; 20), ladite partie mobile (15, 16 ; 20) se trouve dans sa première position radiale et la portion distale (9b) dudit manchon peut librement entrer dans ladite cavité proximale (101) dudit bouchon de piston (100) et en sortir, vers une seconde position axiale, dans laquelle l'extrémité distale (2b, 8 ; 19) de ladite tige (2) applique une force sur ladite partie mobile (15, 16 ; 20) pour déplacer ladite partie vers sa seconde position radiale, et ladite partie mobile (15, 16 ; 20) est capable de s'accrocher à une paroi interne (102) de ladite cavité proximale (101) dudit bouchon de piston (100) pour relier ladite tige de piston (1) audit bouchon de piston (100), ladite tige (2) étant configurée pour se déplacer en translation de sa première position axiale à sa seconde position axiale sous l'effet d'une pression distale exercée sur son extrémité proximale (2a, 3, 4, 4a),
**caractérisée en ce que** la tige de piston comprend en outre
- des moyens de verrouillage libérables (7, 14, 14a ; 25, 25a) configurés pour verrouiller temporairement ladite tige (2) dans sa seconde position axiale.

2. Tige de piston (1) selon la revendication 1, comprenant en outre des moyens de sollicitation (17) configurés pour déplacer automatiquement ladite tige (2) de sa seconde position axiale vers sa première position axiale lorsque lesdits moyens de verrouillage libérables (7, 14, 14a ; 25, 25a) sont libérés.

3. Tige de piston (1) selon la revendication 1 ou 2, dans laquelle l'extrémité proximale (2a) de ladite tige est pourvue d'un capuchon à bouton (3), et une portion proximale (9a) du manchon (9) est pourvue d'une bride radiale (10), et dans laquelle lesdits moyens de sollicitation comprennent un ressort hélicoïdal (17) venant en butée à la fois contre une surface proximale de ladite bride radiale (10) et contre une surface distale dudit capuchon (3), ledit ressort hélicoïdal (17) étant capable de passer d'une position de repos, dans laquelle ladite tige (2) se trouve dans sa première position axiale, à un état contraint, dans lequel ladite tige (2) se trouve dans sa seconde position axiale.

4. Tige de piston (1) selon la revendication 3, dans laquelle ladite tige (2) est configurée pour tourner par rapport audit manchon (9), lesdits moyens de verrouillage libérables comprenant au moins un crochet (7) s'étendant de manière distale à partir dudit capuchon (3) et au moins une rainure circonférentielle partielle (14) située sur ladite bride radiale (10), ledit crochet (7) et ladite rainure circonférentielle (14) étant configurés pour :
- s'engager l'un dans l'autre pour verrouiller ladite tige (2) dans sa seconde position axiale lorsqu'on exerce une pression distale sur ledit capuchon (3) de manière à mettre ledit ressort hélicoïdal (17) à l'état contraint, et qu'on fait tourner en outre ladite tige (2) par rapport audit manchon (9) dans une première direction, et
- se désengager l'un de l'autre pour libérer ladite tige (2) de sa seconde position axiale (5) et la déplacer automatiquement vers sa première position axiale, lorsqu'on fait tourner ladite tige (2) par rapport audit manchon (9) dans le sens opposé à ladite première direction et qu'on relâche ladite pression distale exercée sur ledit capuchon (3).

5. Tige de piston (1) selon l'une quelconque des revendications 1 à 4, dans laquelle la portion distale (9b) dudit manchon (9) est pourvue de deux parties mobiles (20) diamétralement opposées.

6. Tige de piston (1) selon l'une quelconque des revendications 1 à 5, dans laquelle l'extrémité distale (2b) de ladite tige (2) est pourvue d'une partie d'extension (18) configurée pour exercer une force radiale sur ladite partie mobile (20) afin de déplacer ladite partie mobile (20) de sa seconde position radiale vers sa première position radiale lorsque ladite tige (2) revient de sa seconde position axiale vers sa première position axiale.

7. Tige de piston (1) selon la revendication 1 ou 2, comprenant en outre une gaine (22) dimensionnée pour entourer ledit manchon (9) à l'exception de ladite partie mobile (16), ladite gaine (22) ayant un diamètre extérieur sensiblement égal au diamètre extérieur de la portion distale (9b) dudit manchon (9) lorsque ladite partie mobile (16) se trouve dans sa première position radiale.

8. Tige de piston (1) selon la revendication précédente, dans laquelle l'extrémité proximale (2a) de ladite tige (2) est pourvue d'un capuchon (3), et une portion proximale de la gaine (22) est pourvue d'une bride radiale (23), lesdits moyens de sollicitation comprennent un ressort hélicoïdal (17) s'appuyant d'une part sur une surface proximale de ladite bride radiale (23) et d'autre part sur une surface distale dudit capuchon (3), ledit ressort hélicoïdal (17) étant capable de passer d'un état de repos, dans lequel ladite tige (2) se trouve dans sa première position axiale, à un état contraint, dans lequel ladite tige (2) se trouve dans sa seconde position axiale.

9. Tige de piston (1) selon la revendication précédente, dans laquelle ladite tige (2) est configurée pour tourner par rapport à ladite gaine (22), lesdits moyens de verrouillage libérables comprennent au moins un crochet (7) s'étendant de manière distale à partir dudit capuchon (3) et au moins une rainure circonférentielle partielle (25) située sur ladite bride radiale (23), ledit crochet (7) et ladite rainure circonférentielle partielle (25) étant configurés pour :
- s'engager l'un dans l'autre pour verrouiller ladite tige (2) dans sa seconde position axiale lorsqu'on exerce une pression distale sur ledit capuchon (3) de manière à mettre ledit ressort hélicoïdal (17) dans son état contraint, et qu'on fait de plus tourner ladite tige (2) par rapport à ladite gaine (22) dans une première direction, et
- se désengager l'un de l'autre pour libérer ladite tige (2) de sa seconde position axiale (35) et la déplacer automatiquement vers sa première position axiale, lorsque l'on fait tourner ladite tige (2) par rapport à ladite gaine (22) dans le sens opposé à ladite première direction et que l'on relâche ladite pression distale exercée sur ledit capuchon (3).

10. Tige de piston (1) selon l'une quelconque des revendications 1 à 4 et 7 à 9, dans laquelle ladite portion distale (9b) dudit manchon (9) comprend au moins une branche (15) flexible radialement vers l'extérieur, une extrémité distale (16) de ladite branche (15) flexible radialement vers l'extérieur formant ladite partie mobile.

11. Tige de piston (1) selon la revendication 10, dans laquelle ladite portion distale dudit manchon (9) comprend deux branches (15) diamétralement opposées, flexibles radialement vers l'extérieur, une extrémité distale (16) de chaque branche (15) flexible radialement vers l'extérieur formant une partie mobile.

12. Tige de piston (1) selon la revendication 10, dans laquelle ladite portion distale (9b) dudit manchon (9) comprend trois branches (15) flexibles radialement vers l'extérieur, réparties régulièrement sur la circonférence, une extrémité distale (16) de chaque branche (15) flexible radialement vers l'extérieur formant une partie mobile.

13. Tige de piston (1) selon l'une quelconque des revendications 1 à 12, dans laquelle la partie mobile comprend une saillie radiale externe (16).

14. Procédé de liaison d'une tige de piston (1) selon l'une quelconque des revendications 1 à 13 à la cavité proximale (101) d'un bouchon de piston (100) d'un dispositif d'injection, ledit procédé comprenant les étapes suivantes :
- la fourniture de la tige de piston (1) avec la tige (2) dans sa première position axiale,
- l'insertion de la portion distale (9b) du manchon (9) à l'intérieur de la cavité proximale (101) du bouchon de piston (100),
- l'application d'une pression distale sur l'extrémité proximale (2a, 3, 4, 4a) de la tige (2) afin de guider la tige (2) vers sa seconde position axiale,
- l'activation des moyens de verrouillage (7, 14, 14a ; 25, 25a) pour verrouiller temporairement la tige (2) dans sa seconde position axiale.

15. Procédé de détachement d'une tige de piston (1) selon l'une quelconque des revendications 1 à 13, qui a été reliée à la cavité proximale (101) d'un bouchon de piston (100) selon le procédé de la revendication 13, comprenant les étapes suivantes :
- la désactivation des moyens de verrouillage (7, 14, 14a ; 25, 25a),
- le relâchement de toute pression distale appliquée sur l'extrémité proximale (2a, 3, 4, 4a) de ladite tige (2) afin de mettre ladite tige (2) dans sa première position axiale,
- le retrait de la partie distale (9b) du manchon (9) de la cavité proximale (101) du bouchon de piston (100).
